## Europäisches Patentamt

## European Patent Office

## Office européen des brevets

(19)

(11) Veröffentlichungsnummer : **0 165 285 B1**

(12) **EUROPÄISCHE PATENTSCHRIFT**

(45) Veröffentlichungstag der Patentschrift :
07.09.88

(51) Int. Cl.⁴ : **A 61 N 1/42, A 61 N 1/36, A 61 N 5/02**

(21) Anmeldenummer : 85900161.2

(22) Anmeldetag : 07.12.84

(86) Internationale Anmeldenummer :
PCT/HU 84/00059

(87) Internationale Veröffentlichungsnummer :
WO/8502547 (20.06.85 Gazette 85/14)

(54) **Elektrische Schaltung für ein medizinisches Gerät zum Erzeugen eines sich ändernden Magnetfeldes.**

(30) Priorität : 08.12.83 HU 419483

(43) Veröffentlichungstag der Anmeldung :
27.12.85 Patentblatt 85/52

(45) Bekanntmachung des Hinweises auf die Patenterteilung : 07.09.88 Patentblatt 88/36

(84) Benannte Vertragsstaaten :
AT DE

(56) Entgegenhaltungen :
EP-A- 0 057 048
AT-B- 287 178
CH-A- 633 190
DD-A- 24 660
DD-A- 82 175
DE-A- 2 533 360
DE-A- 2 803 084
FR-A- 2 440 202
JP-B-58 008 253
US-A- 3 299 892

(73) Patentinhaber : GYÜLING, Zoltán
Géza u. 90
H-8000 Székesfehérvár (HU)

SCHINDLER, Rozsa
Géza u. 90
H-8000 Székesfehérvár (HU)

BORDACS, Ferenc
Vörös hadsereg utja 272
H-8000 Székesfehérvár (HU)

MAROS, Jânos
Jokai u. 4
H-8000 Székesfehérvár (HU)

SCHUBERT, Zsuzsa
Ady E. u. 11
H-8000 Székesfehérvár (HU)

(72) Erfinder : GYÜLING, Zoltán
Géza u. 90
H-8000 Székesfehérvár (HU)
Erfinder : SCHINDLER, Rozsa
Géza u. 90
H-8000 Székesfehérvár (HU)
Erfinder : BORDACS, Ferenc
Vörös hadsereg utja 272
H-8000 Székesfehérvár (HU)
Erfinder : MAROS, Jânos
Jokai u. 4
H-8000 Székesfehérvár (HU)
Erfinder : SCHUBERT, Zsuzsa
Ady E. u. 11
H-8000 Székesfehérvár (HU)

(74) Vertreter : **Patentanwälte Viering & Jentschura**
**Steinsdorfstrasse 6**
**D-8000 München 22 (DE)**

**Beschreibung**

Die Erfindung betrifft eine elektrische Schaltung für ein medizinisches Gerät zum Erzeugen eines sich zeitlich ändernden Magnetfeldes, mit einer Behandlungsspule, einem Impulsgenerator, einem Leistungsverstärker und einer Speiseeinheit.

Die Wirkung eines Magnetfeldes auf den menschlichen Organismus ist seit Urzeiten bekannt. Im Altertum wie auch im Mittelalter wurden zur Heilung natürliche magnetische Substanzen verwendet.

Als Ergebnis der elektrotechnischen Entwicklung wurden in zweiter Hälfte des XIX. Jahrhunderts die ersten Vorstellungen bezüglich einer elektromagnetischen Therapie entwickelt. In der DE-A-96044 aus dem Jahr 1869 wurde eine beinahe den ganzen Körper eines Kranken umgebende Spule vorgeschlagen.

Das Interesse an der elektromagnetischen Therapie und auch die intensive wissenschaftliche Untersuchung ihrer Heilwirkung haben in dem letzten Jahrzehnt bedeutend zugenommen. Bei sogenannten nichtinvasiven Verfahren wird ein Feld in der Umgebung des kranken Körperteils mit einzelnen Impulsen von 10-20 ms Basis eine für bestimmte Zeit erregt. Die Impulse haben gegebenenfalls sinusförmigen Verlauf.

In der JP-A-588253 wird eine Schaltung vorgeschlagen, bei der die Intervalle einer Niederfrequenzimpulsreihe zwischen den einzelnen Impulsen stufenweise vermindert werden.

Der Nachteil der bisherigen Schaltungen zur Erzeugung therapeutisch wirksamer Magnetfelder für nichtinvasive Heilverfahren besteht darin, daß die Genesung eines mit diesen Magnetfeldern behandelten Kranken erst nach längerer Zeit, durchschnittlich nach 5-10 Behandlungen beginnt. Bei den sogenannten invasiven Verfahren wird neben der Erzeugung eines Feldes für die Dauer der Behandlung eine Hilfselektrode in den kranken Körperteil eingesetzt. Diese Verfahren sind wesentlich wirksamer als die ersten. Infolge der Notwendigkeit des Einsetzens von Hilfselektroden können aber auch diese Verfahren nicht als perfekt angesehen werden.

Durch die Erfindung wird die Aufgabe gelöst, eine elektrische Schaltung mit den Merkmalen aus dem Oberbegriff des Anspruchs 1 zu schaffen, die ein sich zeitlich änderndes Magnetfeld erzeugen kann, dessen medizinische Wirksamkeit gegenüber den mit Hilfe der bekannten Schaltungen erzeugten Magnetfeldern gesteigert ist und das mehr therapeutische Anwendungsmöglichkeiten bietet.

Dies wird erfindungsgemäß dadurch erreicht, daß an ein Ende der Behandlungsspule über einen DC/DC Umformer die Speiseeinheit und an das andere Ende über einen Leistungsverstärker ein Impulsgenerator mit konstantem Tastverhältnis und änderbarer Frequenz angeschlossen ist, und zwischen dem Impulsgenerator und dem Leistungsverstärker mindestens ein, vorzugsweise zwei Impulsreihengenerator/en geschaltet ist/sind.

Bevorzugte Ausgestaltungen der erfindung sind Gegenstand der Unteransprüche.

Die Erfindung wird im weiteren an Hand der Zeichnung beschrieben.

Es zeigt:
Figur 1 das Blockdiagramm der Schaltung,
Figur 2 den Schaltplan eines zweckmäßigen Ausführungsbeispiels,
Figur 3 die Kennlinien bestimmter Teile der Schaltung.

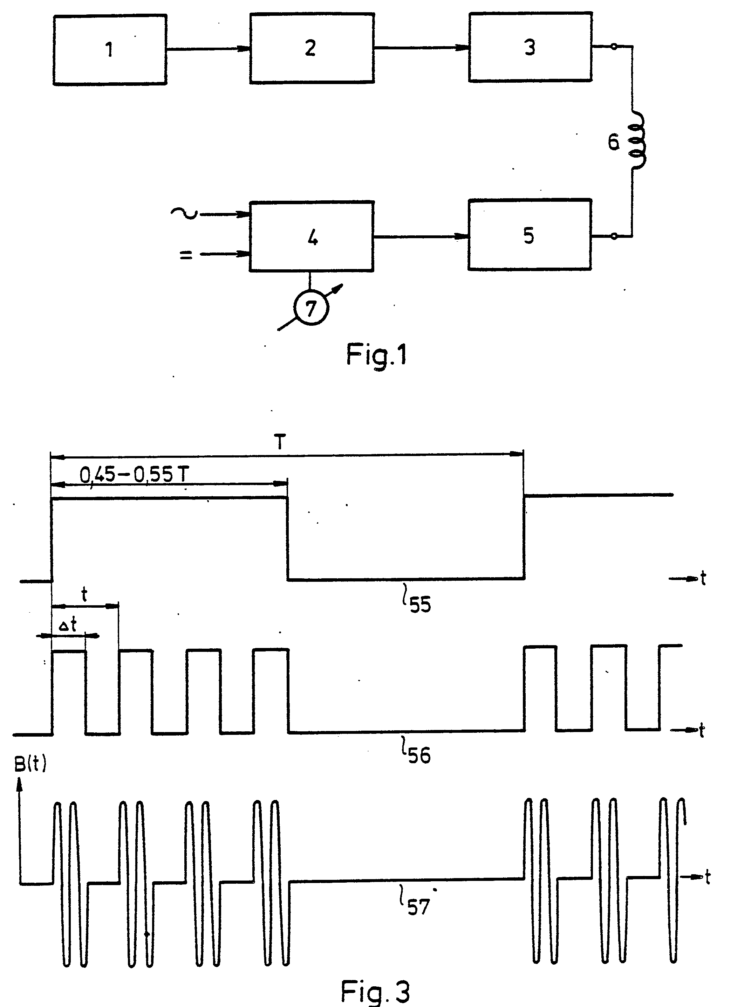

In Figur 1 ist das Blockdiagramm einer bevorzugten Ausführungsform der erfindungsgemäßen Schaltung dargestellt. Eine als stabilisierte Spannungseinheit mit änderbarer Spannung ausgestaltete Speiseeinheit ist über einen DC/DC Umformer 5 mit einem Ende der die Umgebung des kranken Körperteils erregenden Behandlungsspule 6 verbunden. Bei der dargestellten Ausführungsform ist die Speiseeinheit 4 zweckmäßigerweise mit einem digitalen Anzeigeeinheit 7 versehen. Die Speiseeinheit 4 kann sowohl mit Netzstrom als auch über einen Speicher betrieben werden.

Zwei Generatoren 1, 2 sind über einen Leistungsverstärker 3 mit dem anderen Ende der Behandlungsspule 6 verbunden. Zwischen einem Impulsgenerator 1 mit konstantem Tastverhältnis (etwa 0,45-0,55) und veränderbarer Niederfrequenz und einem Leistungsverstärker 3 ist ein Impulsreihengenerator 2 angeschlossen. Der Impulsgenerator 1 ist zweckmäßigerweise ein Rechteckimpulse lieferndes Gerät, dessen Impulsreihen die Impulsreihen des Generators 2 als Fensterfunktion überlagern.

Der Impulsreihengenerator 2 ist eigentlich aus zwei Einheiten zusammengesetzt, von denen jede ein selbstständiger Impulsreihengenerator ist.

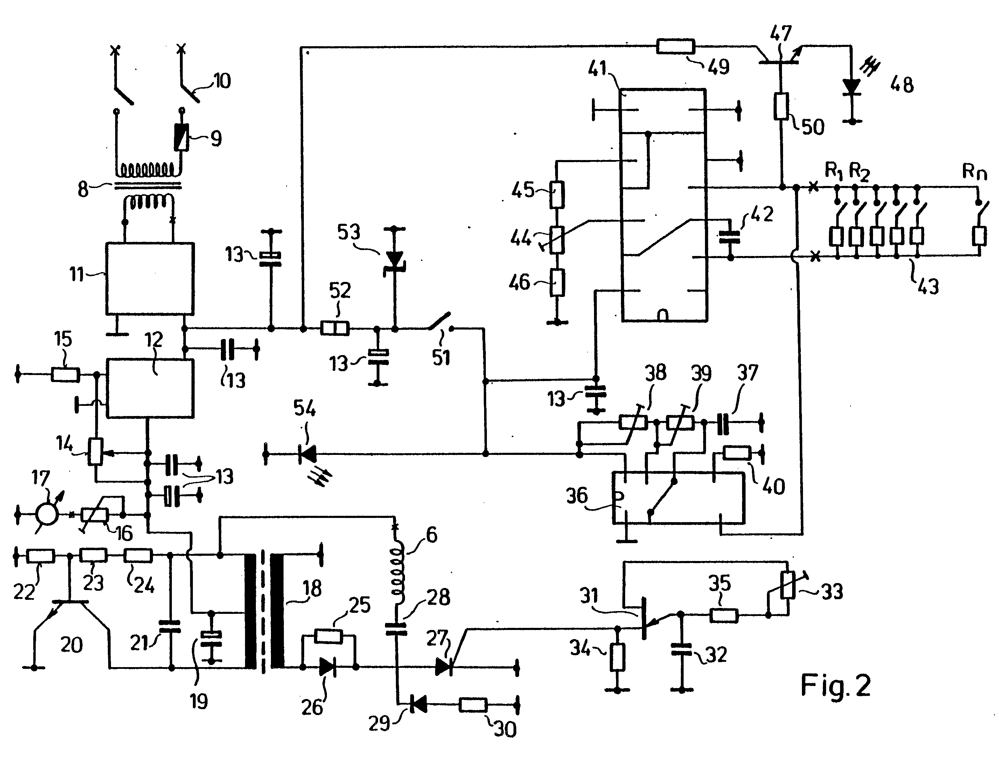

In Figur 2 ist ein schematischer Schaltplan eines zweckmäßigen Ausführungsbeispiels der erfindungsgemäßen Schaltung dargestellt. Die Primärspule des Transformators 8 der Speiseeinheit 4 ist über eine Sicherung 9 und einen Schalter 10 an das Netz angeschlossen.

Die Sekundärspule des Transformators 8 ist an einen Gleichrichter 11 angeschlossen. Der Ausgang des Gleichrichters ist in zwei Leitungen geteilt, von denen eine an einen als integrierte Schaltung ausgestalteten Stabilisator 12 angeschlossen ist.

Es soll erwähnt werden, daß am erwähnten Teilungspunkt eine in der Zeichnung nicht dargestellt Gleichstromquelle, z. B. ein 24 V Akkumulator angeschlossen werden kann.

Der Gleichrichter 11 und der Stabilisator 12 sind mit je einem Filterkondensator 13 versehen. Die weiteren in der Zeichnung dargestellten Filterkondensatoren 13 der schaltung werden in der Beschreibung nicht erwähnt.

Der Stabilisator 12 ist mit einem aus einem Potentiometer 14 und aus einem Widerstand 15 zusammengestellten Dämpfungsglied versehen, das zur Einstellung der Spannung dient.

An dem Ausgang des Stabilisators 12 ist einerseits das Eichpotentiometer 16 eines Induktionsmessgeräts 17 und andererseits die Primärspule des zweckmäßigerweise als Toroidtransformator ausgestalteten Transformators 18 angeschlossen. Der Transformator 18 ist über einen Kondensator 19 geerdet.

Die Primärspule ist mit dem aus einem Transistor 20, einem Kondensator 21 und den Widerständen 22-24 zusammengestellten Relaxationsleistungsoszillator verbunden. Der Ausgang des Schwingkreises ist an einem Pol des Behandlungsspule angeschlossen.

An dem Ausgang der Sekundärspule des Transformators 18 befindet sich eine Gleichrichterdiode 26 mit Schutzwiderstand 25, deren Ausgang mit dem aus einem Thyristor 27, einem Kondensator 28 und einer Behandlungsspule 6 bestehenden Stromkreis verbunden ist. Der Thyristor 27 ist mit einem aus einer Diode 29 und aus einem Widerstand 30 bestehenden Schutz versehen.

Der Thyristor 27 wird von einem Stromkreis gesteuert, der zweckmäßigerweise aus einem Unijunction Transistor 31, einem Kondensator 32, einem Potentiometer 33 und den Widerständen 34-35 besteht.

An den Eingang dieses Stromkreises ist ein analoger integrierter Zeitgeberstromkreis angeschlossen, welcher einerseits ein aus einem Kondensator 37 und den Potentiometern 38-39 zusammengesetztes Däpfungsglied und andererseits einen Schutzwiderstand 40 enthält.

Das Frequenzregelungselement des als analoger integrierter Stromkreis ausgestalteten Rechteckimpulsgenerators 41 besteht zweckmäßigerweise aus einem Kondensator 42 und einem Potentiometer 43.

Der analoge integrierte Zeitgeberstormkreis 36 wird von dem Ausgang des Generators 41 gesteuert.

Der Generator 41 hat eine aus einem Potentiometer 44 und aus den Widerständen 45-46 zusammengestellte Kette.

Der Generator 41 hat zweckmäßigerweise auch einen den aktivierten Zustand des magnetischen Feldes anzeigenden Stromkreis, welcher aus einem Transistor 47, einer LED 48 und aus den verstellbaren Widerständen 49-50 zusammengesetzt ist.

In dem Speisestromkreis des Generators 41 ist außer einem Schalter 51 der einen Widerstand 52 und eine Zenerdiode 53 enthaltende Stabilisator enthalten und dieser ist zweckmäßigerweise mit einer LED 54 ergänzt.

Im weiteren wird der Betrieb der erfindungsgemäßen Schaltung eingehend beschrieben.

In Figur 3 sind die den Betrieb der einzelen Konstruktionselemente kennzeichenenden Kurven gezeigt.

Der Relaxationsleistungsoszillator wird über den Schalter 10 mit Strom versorgt, und die medizinischen indizierte Induktion wird mit dem Potentiometer 14 zwischen 20 und $100 \times 10^{-4}$ T eingestellt.

In dem nächsten Schritt wird die Basisfrequenz mit dem Potentiometer 43 zwischen 2 und 50 Hz eingestellt.

Die Behandlungsspule 6 wird an dem zu behandelnden Körperteil oder in seiner Nähe angeordnet und durch Schließen des Schalters 51 werden der Impulsgenerator 1 und zwei Einheiten des Impulsreihengenerators 2 in Betrieb gesetzt.

Die Ausgangssignale des Impulsgenerators 1 und des dem ersten Impulsreihengenerator 2 entsprechenden analog integrieten Zeitgeberstromkreises sind in Figur 3 durch die Kurve 55 bzw. 56 symbolisiert. Das Signal an der Behandlungsspule 6 ist durch die Kurve 57 dargestellt.

Die Dauer t und die effektive Zeit $\Delta$ t wenigstens einer der während des Zeitraums 0 bis 0,45-0,55 T der veränderbaren Widerholungsperiode T auftretenden Perioden kann durch Regulierung des Dämpfungsgliedes des Stromkreises eingestellt werden, während die Form der während $\Delta$ t zustandekommenden zweiten Impulsreihe mit dem Potentiometer 33 eingestellt wird. In dieser letzten Impulsreihe sind wenigstens zwei, zweckmäßigerweise mehrere Impulse vorhanden.

Der Vorteil der erfindungsgemäßen Schaltung ist, daß mit ihr therapeutisch wirksame Magnetfelder erzeugt werden können, die auf dem Gebiet der Orthopädie, der Behandlung der Sportverletzungen, der Chirurgie, der Geriatrie, der Stromatologie, der Augenheilkunde, der inneren Heilkunde, der Otorhinolarynologie, der Rehabilitation und der Neurologie gleichfalls therapeutisch verwendet werden können. Besonders wichtig ist die Tatsache, daß diese Magnetfelder ebenfalls zur Heilung der Multiplen Sklerose geeignet sind.

**Patentansprüche**

1. Elektrische Schaltung für ein medizinisches Gerät zum Erzeugen eines sich zeitlich ändernden Magnetfeldes, in der eine Behandlungsspule (6), ein Impulsgenerator (1), ein Leistungsverstärker (3) und eine Speiseeinheit (4) eingebaut sind, dadurch gekennzeichnet, daß an ein Ende der Behandlungspule (6) über einen DC/DC Umformer (5) die Speiseeinheit und an das andere Ende über den Leistungsverstärker (3) ein Impulsgenerator (1) mit konstantem Tastverhältnis und änderbarer Frequenz angeschlossen ist und zwischen dem Impulsgenerator (1) und dem Leistungsverstärker (3) mindestens ein, vorzugsweise zwei Impulsreihengenerator/en (2) geschaltet ist/sind.

2. Schaltungsanordnung nach Anspruch 1, dadurch gekennzeichnet, daß der Impulsgenerator (1) ein Rechteckimpulsgenerator ist.

3. Schaltungsanordnung nach Anspruch 1 oder 2, dadurch gekennzeichnet, daß die Impulsreihen des Impulsreihengenerators (2) von einer Fensterfunktion überlagert werden.

4. Schaltungsanordnung nach einem der Ansprüche 1-3, dadurch gekennzeichnet, daß die Speiseeinheit eine stabilisierte Einheit änderbarer Spannung ist.

5. Schaltungsanordnung nach einem der Ansprüche 1-4, dadurch gekennzeichnet, daß die Speiseeinheit (4) mit einem digitalen Anzeigegerät (7) versehen ist.

## Claims

1. Electrical circuit for medical apparatus for producing a varying magnetic field, having a treatment coil (6), a pulse generator (1), a power amplifier (3) and a feeding unit (4), characterized in that the feeding unit is connected to one end of the treatment coil (6) via a DC/DC-transformer (5) and that a pulse generator (1) having a constant pulse duty factor and being changeable in frequency is connected to the other end of the treatment coil (6) via a power amplifier (3), and that at least one pulse series generator, preferably two pulse series generators (2), are circuited between the pulse generator (1) and the power amplifier (3).

2. Electrical circuit according to claim 1, characterized in that the pulse generator (1) is a square pulse generator.

3. Electrical circuit according to claim 1 or 2, characterized in that the pulse series of the pulse generator (2) is superimposed by a window function.

4. Electrical circuit according to one of claims 1 to 3, characterized in that the feeding unit is a stabilized unit of changeable voltage.

5. Electrical circuit according to one of claims 1 to 4, characterized in that the feeding unit (4) is provided with a digital display device.

## Revendications

1. Circuit électrique pour un appapreil médical produisant un champ magnétique variable, avec un self de traitement (6), un générateur d'impulsions (1), un amplificateur de puissance (3) et un élément d'alimentation (4), caractérisé en ce que l'élément d'alimentation est relié à un bout du self de traitement (6) via un transformateur DC/DC (5) et qu'un générateur d'impulsions (1) avec un rapport durée d'impulsions constant et ayant une fréquence changeable est relié à l'autre bout du self du traitement via un amplificateur de puissance (3), et qu'au moins un générateur d'impulsions de série, préférablement deux générateurs d'impulsions de série (2) est relié/sont reliés entre le générateur d'impulsions (1) et l'amplificateur de puissance (3).

2. Circuit électrique selon la revendication 1, caractérisé en ce que le générateur d'impulsions (1) est un générateur d'impulsions rectangulaire.

3. Circuit électrique selon l'une des revendications 1 ou 2, caractérisé en ce que la série d'impulsions du générateur d'impulsions (2) est superposée d'une fonction de fenêtre.

4. Circuit électrique selon l'une des revendications 1 à 3, caractérisé en ce que l'élément d'alimentation est un élément stabilisé de tension changeable.

5. Circuit électrique selon l'une des revendications 1 à 4, caractérisé en ce que l'élément d'alimentation (4) est muni d'un appareil indicateur (7) digital.

0 165 285

Fig.1

Fig.3

1

Fig. 2